# EUROPEAN PATENT APPLICATION

(11) **EP 3 763 701 A1**
(43) Date of publication of application: **13.01.2021**
(21) Application number: 19185475.1
(22) Date of filing: 10.07.2019
(51) Int. Cl.: C07C 309/04

(54) **IMPROVED PROCESS FOR THE PURIFICATION OF METHANE SULFONIC ACID**

(71) Applicant: Grillo-Werke AG, 47169 Duisburg (DE)
(72) Inventor: Ott, Timo, 47169 Duisburg (DE)
(74) Representative: BASF IP Association

(57) **Abstract**

The present application discloses a process to provide purified alkane sulfonic acid comprising the following steps:
a) providing a liquid mixture comprising methane sulfonic acid,
b) adjusting the mixture such that the concentration of methane sulfonic acid is at least 30 % by weight, based on the total amount of the mixture,
c) distillation or rectification of the mixture.

## Description

The present invention refers to an improved process for the purification of methane sulfonic acid, in which the concentration of methane sulfonic acid in a mixture is set such that less energy is needed for the purification by distillation.

Alkanesulfonic acids are organic acids that can reach a similar acid strength as that of inorganic mineral acids, for example, sulfuric acid. However, in contrast to usual mineral acids such as sulfuric and nitric acids, the sulfonic acids are non-oxidizing and do not give off vapors that are harmful to health, as can be observed with hydrochloric and nitric acids. Further, many sulfonic acids, for example, methane sulfonic acid, are biologically degradable. The applications of sulfonic acids are many, for example, in cleaning agents, surfactants, as catalysts, and in organic synthesis, pharmaceutical chemistry, for example, as protective groups. The salts of sulfonic acids are employed, for example, as surfactants, for example, sodium dodecylsulfonate, or in the electroplating industry, especially as tin, zinc, silver, lead and indium, but also other metal alkylsulfonates. The very high solubility of alkyl sulfonates plays an important role, in particular. Further, no harmful gases are formed in electrolysis, and the use of toxic compounds, for example, cyanide, which is common in many cases, is dispensed with.

The structurally simplest representative of alkane sulfonic acids is methane sulfonic acid. Different methods for the synthesis are described in the prior art, for example in US 2,493,038, US 2005/0070614, WO 2007/136425 A2, EP 3071549 A1. In the prior art, methane and SO₃ are reacting with each other. The resulting reaction mixture comprises methane sulfonic acid as well as SO₃ as non-reacted educt. This SO₃ should be removed prior to distillation, as otherwise side products may occur (Robinson and Silberberg: "The reaction of methanesulfonic acid with sulfur trioxide", Canadian Journal of Chemistry, Vol. 44 (1966), 1437ff). Respective removal is for example disclosed in WO 2018/208701 A1 or European patent application 19176382.0. WO 2018/208701 A1 and European patent application 19176382.0 both disclose distillation as final purification step.

The distillation of methane sulfonic acid from a reaction mixture has to be controlled carefully. The conditions are strongly dependent on the composition of the reaction mixture. Surprisingly it was found that it is possible to provide pure methane sulfonic acid with low level impurities by increasing the concentration of methane sulfonic acid in a liquid mixture prior to distillation.

In a first embodiment, the problem of the present application is thus solved by a process to provide purified alkane sulfonic acid comprising the following steps:
a) providing a liquid mixture comprising methane sulfonic acid,
b) adjusting the mixture such that the concentration of methane sulfonic acid is at least 30 % by weight, based on the total amount of the mixture,
c) distillation or rectification of the mixture.

In a preferred embodiment, the process further comprises the step
d) purifying the methane sulfonic acid obtained from step c) by adsorption methods.

The inventors of the present application surprisingly found that it helps to improve the quality of the methane sulfonic acid if the concentration of methane sulfonic acid in the reaction mixture which is distilled is above 30 % by weight, based on the total weight of the mixture. The higher the concentration of methane sulfonic acid in the mixture, the lower is the energy need for the distillation, as the distillation can be performed at lower temperatures compared to temperatures needed for lower concentrations of methane sulfonic acid. If a mixture is used from a process to produce methane sulfonic acid from CH₄ and SO₃, the resulting reaction mixture usually comprises methane sulfonic acid in a concentration of about 5 % by weight to 10 % by weight. If the concentration of methane sulfonic acid is increased to about 40 % by weight to 50 % by weight, the temperature for the distillation can be reduced by 30 Calvin or even 35 Calvin (at the same pressure of below 10 mbar). The higher the concentration of methane sulfonic acid, the lower the distillation temperature.

Preferably, the concentration of methane sulfonic acid in the mixture is therefore in a range from 35 % by weight to 95 % by weight or to 90 % by weight, especially from 40 % by weight to 85 % by weight, preferred from 45 % by weight to 80 % by weight, advantageously from 50 % by weight to 75 % by weight, especially preferred from 55 % by weight to 70 % by weight or from 60 % by weight to 65 % by weight. The amounts of weight always refer to the total weight of the mixture, which is 100 % by weight.

Thus, in cases where the amount of methane sulfonic acid in the reaction mixture is below the above-mentioned value, it is within the scope of the invention to increase the concentration of methane sulfonic acid in the reaction mixture, meaning at the same time that the concentration of other compounds in the mixture is reduced. Thus, the process of the present invention not only describes an increase of methane sulfonic acid but also decrease of certain types of impurities, especially sulfate containing impurities.

A mixture within the scope of the present application can be a mixture resulting from a process of producing methane sulfonic acid. Different ways of producing methane sulfonic acid are known. A reaction mixture within the meaning of the present application is especially a mixture resulting from a method of producing methane sulfonic acid from methane and sulfur trioxide. Preferably, it is a method as disclosed in WO 2007/136425 A2 or a method as disclosed in EP 3071549 A1, the content of which is enclosed herein in its entirety.

A mixture within the meaning of the present application could also be a mixture which is obtained after methane sulfonic acid was used in different technical fields. For example, in processes where methane sulfonic acid is used as solvent or as kind of catalyst, it is of interest to kind of recycle the methane sulfonic acid again. This recycling can be performed by distillation. To receive also here high quality of methane sulfonic acid after distillation, it is also advisable to increase the amount of methane sulfonic acid in the mixture prior to distillation. Therefore, the mixture within the meaning of the present application is not only a reaction mixture obtained from a process to produce methane sulfonic acid, but any reaction mixture comprising methane sulfonic acid. Liquid within the meaning of the present invention means liquid under ambient conditions (20 °C and 1 bar pressure).

After distillation of the mixture, methane sulfonic acid is obtained as product. The methane sulfonic acid might still comprise impurities, especially sulphate impurities. During distillation, methane sulfonic acid anhydride is often formed. The value thereof should be 100 ppm or less in the final product. Thus, there may be still the need to reduce the amount of methyl sulfonic acid anhydride below this value. Also, other impurities may be present, which should be reduced if possible. A respective reduction can be obtained by adsorption processes.

Methane sulfonic acid anhydride is formed during distillation. The higher the temperature during distillation, the higher the concentration of the anhydride formed during distillation. The anhydride itself is unstable and decays to different impurities. The process of the present invention now enables to perform the distillation at lower temperatures due to the higher concentration of methane sulfonic acid in the mixture. Thus, also the concentration of methyl sulfonic acid anhydride and other impurities in the final product is reduced compared to conventional distillation processes.

To obtain a pure methane sulfonic acid, it is within the scope of the present invention to either increase the concentration of methane sulfonic acid and decrease the concentration of impurities prior to distillation or to purify the methane sulfonic acid obtained after the distillation. It is also within the scope to perform both, namely first increasing the concentration of methane sulfonic acid in the mixture and further purify the methane sulfonic acid obtained after distillation.

The increase of the methane sulfonic acid or decrease of sulphate impurities in step b) of the present invention is preferably performed by melt crystallization and/or sorption.

If melt crystallization is used to increase the concentration of methane sulfonic acid, it is essential that the reaction mixture comprises either some concentration s of water or excess of sulfur trioxide should be present. In case where water and methane sulfonic acid are present, it is assumed that the monohydrate of methane sulfonic acid is formed, which can be crystallized at temperatures around -5 °C to -50 °C, especially at -10 °C to -25 °C. The molar ratio between MSA and water in the reaction mixture should thus be in the range between 1:5 to 1:1, preferably about 1:2 to 1:4 or 1:3 (always meaning the molar ratio methane sulfonic acid to water).

Alternatively, if the crystallization cannot be performed as the addition of water shall be avoided, it is also possible to add some excess of sulfur trioxide, enabling again the formation of crystals at a temperature between -5 °C to - 15 °C. here, the molar ratio between methane sulfonic acid and sulfur trioxide is preferably within the range of from 1:3 to 1:1 or 1:2.

Surprisingly, it was found that an increase of methane sulfonic acid of about 2-20% by weight was performed. The amount means here absolute values. For example, if the original reaction mixture comprises 30% by weight of methane sulfonic acid, it was possible to increase the concentration of methane sulfonic acid to be close to 50% by weight. This would mean an increase of about 20% by weight in absolute amount.

The increase of the methane sulfonic acid was observed in case where the crystals are separated from the liquid after the crystallization. The melt of the crystals can then be used for the distillation step. At the same time, this also means that in the liquid phase, the concentration of methane sulfonic acid was decreased.

The increase of methane sulfonic acid or decrease of especially sulphate impurities may also be performed by adsorption processes, especially by physisorption and/or chemisorption. The adsorption is preferably performed with a membrane and/or a porous structure. Suitable membranes are ceramic nano-filtration membranes. In cases where methane sulfonic acid is produced by reacting methane sulfur trioxide, these membranes may have a cut off from about 200 Dalton. Preferably, the reaction mixture is water-free, as water-free systems enable a higher increase of the concentration of methane sulfonic acid by ceramic membranes. The increase of concentration of methane sulfonic aid was about 1% by weight to 5% by weight in absolute values (as defined above).

A physisorption processes can also be used to increase the concentration of methane sulfonic acid. Respective porous structures can be provided as solid additives. Their porous size is to be adapted to the size of the impurities which should be reduced. At the same time, the size of methane sulfonic acid kept in mind so that the methane sulfonic acid is not adsorbed by the porous structure.

Suitable porous structures are molecular sieves, silicon oxide, aluminium oxide or zeolites. In case where the reaction mixture is obtained from a process of preparing methane sulfonic acid based on contacting methane with sulfur trioxide, the porous size of the porous structure, especially of the molecular sieve, is within the range from 3 Å to 10 Å, especially within a range from 3 Å to 5 Å.

If by the above-mentioned methods, the concentration of methane sulfonic acid in the reaction mixture is increased to a concentration, which is suitable to perform the distillation, this is performed with at least one distillation column. This distillation column can be adapted in its stages. Thus, for example, a reaction mixture comprising methane sulfonic acid, sulfuric acid, water and SO₃ is provided as mixture. In a first step, the concentration of methane sulfonic acid in the reaction mixture is increased by any of the above described methods. This reaction mixture is then provided into a distillation means. It is possible to use the product obtained from the distillation means again to provide it in the reaction mixture obtained after increasing the concentration of methane sulfonic acid to further increase the concentration in the mixture which is provided into the distillation means.

It is further preferred, that the reaction mixture is provided directly into the distillation tower. In a preferred embodiment, there is a heating element provided prior to the inlet, where the reaction mixture is added into the distillation tower. This heating element enables a constant temperature in the distillation column so that a good separation of methane sulfonic acid from other compounds of the reaction mixture can be obtained.

In the bottom of the distillation means, the still, there is a solution comprising methane sulfonic acid in lower concentrations. It is of course possible, to circulate this liquid and also bring it into contact with the heating element to have a temperature as constant as possible, which is about 200 °C.

The distillation column is heated and isolated, to ensure that the temperature inside the column is as constant as possible. The material of the distillation means can be any material which is resistant against the acidity of methane sulfonic acid and any other acid which may be present in the reaction mixture. Preferably, the material is selected from glass, Teflon, silicon carbide, ceramic materials, porcelain, especially glazed porcelain, emaille, stainless steel, especially with an emaille coating. The material has to be selected such that there is no direct contact between a metal and the reaction mixture, as methane sulfonic acid and may be other assets can react with different kinds of metals or metals can catalyse any unwanted side reaction.

When heating the distillation column and the still, it must be controlled that the temperature is always homogenously distributed. It should be avoided that at certain positions high temperatures are rising. This can be performed by mixing, e.g. by stirring, or circulating the mixture in the still.

The distillation may be performed within one distillation column. It is also within the scope of the invention that 2, 3 or more distillation columns are used. The length of the distillation column can be adjusted such that in the still there is only sulfuric acid present. This enables an extremely high temperature of the still but has the disadvantage of lot of space is needed for such a process.

Thus, there are several parameters in the distillation which can be adapted to obtain a methane sulfonic acid which as pure as possible. At the same time the formation of side products can be controlled.

Nevertheless, it is still possible that during distillation side products are formed. Especially for the removal of such side products but also of other impurities which could not be removed by distillation, a purification step after distillation may be performed. This additional purification step is preferably performed as physorption and/or chemisorption process. A porous structure, especially activated carbon, a molecular sieve, silicon oxide, aluminium oxide and/or zeolite may be used as physisorption of sulphate impurities. Again, as already discussed for the increasing step, the size of the pores of the porous structure has to be adapted such that the impurity is adsorbed at the porous structure, but the methane sulfonic acid is not.

Preferably, the purification after the distillation is performed via chemisorption. Here a chemical compound can be used, having a functionalization forming a nucleophilic group. This nucleophilic group is able to form anions which then react with side products, especially with side products comprising highly reactive methyl residues.

The main problem during distillation of methane sulfonic acid is that methane sulfonic acid methyl ester (MMS) is formed. For example, if an amino functionalized polymer is used, MMS reacts with the amino group of the polymer leading to the formation of methyl amine which is fixed at the polymer, and methane sulfonic acid.

Suitable polymers are for example polyethylene, polypropylene and/or polystyrene. These polymers may have for example an amino functionalization or a hydroxy functionalization. This means of course, that there is not necessarily only one functional group present in the polymer but several may be present. It is also possible that a polymer comprises amino and hydroxy-functional groups.

As MMS is larger than methane sulfonic acid, also a molecular sieve, activated carbon or other porous structure defined above may be used to reduce the concentration of MMS and allow to provide pure methane sulfonic acid.

## Claims

1. Process to provide purified alkane sulfonic acid comprising the following steps:
a) providing a liquid mixture comprising methane sulfonic acid,
b) adjusting the mixture such that the concentration of methane sulfonic acid is at least 30 % by weight, based on the total amount of the mixture,
c) distillation or rectification of the mixture.

2. Process according to claim 1, wherein the process further comprises the step
d) purifying the methane sulfonic acid obtained from step c) by adsorption methods.

3. Process according to claim 1 or 2, wherein the increase in step b) is performed by melt crystallisation and/or adsorption.

4. Process according to claim 3, wherein the adsorption is a physisorption and/or chemisorption.

5. Process according to claim 3, wherein the adsorption is performed with a membrane and/or a porous structure, especially a molecular sieve.

6. Process according to any of claims 1 to 5, wherein the mixture comprises methane sulfonic acid, SO₃, methane and sulfuric acid.

7. Process according to any of claims 1 to 6, wherein the purification in step d) is performed as physisorption and/or chemisorption.

8. Process according to claim 7, wherein the physisorption is performed at a porous structure, especially activated carbon, molecular sieve, silicon oxide, aluminum oxide and/or zeolite.

9. Process according to claim 7, wherein the chemisorption is performed with a functionalized polymer, especially with a polymer having at least one amino and/or hydroxy-functional group.

10. Process according to claims 9, wherein the polymer is polyethylene, polypropylene and/or polystyrene.
